# EUROPÄISCHE PATENTSCHRIFT

(11) **EP 0 643 042 B1**
(45) Veröffentlichungstag und Bekanntmachung des Hinweises auf die Patenterteilung: **17.07.1996**
(21) Anmeldenummer: 94113844.8
(22) Anmeldetag: 03.09.1994
(51) Int. Cl.: C07C 263/18, C07C 265/14

(54) **Lagerstabile, nach phosgenfreien Verfahren erhältliche Polyisocyanatzusammensetzungen, ein Verfahren zu ihrer Herstellung und ihre Verwendung**
Storage-stable polyisocyanate mixtures prepared by phosgen-free processes, a process for their preparation and their use
Mélanges de polyisocyanates stables préparés par un procédé sans phosgène, un procédé pour leur préparation et leur emploi

(30) Priorität: 13.09.1993 DE 4331083
(43) Veröffentlichungstag der Anmeldung: 15.03.1995
(73) Patentinhaber: BASF Aktiengesellschaft, D-67063 Ludwigshafen (DE)
(72) Erfinder: Laqua, Gerhard, Dr., D-68167 Mannheim (DE); Merger, Franz, Dr., D-67227 Frankenthal (DE); Witzel, Tom, Dr., D-67069 Ludwigshafen (DE); Siebenhaar, Ursula, Dr., D-67069 Ludwigshafen (DE); Agar, David, Dr., D-68161 Mannheim (DE)

(56) Entgegenhaltungen:
- EP-A- 0 203 874
- EP-A- 0 355 443
- EP-A- 0 445 608
- EP-A- 0 531 803
- DATABASE WPI Section Ch, Week 9333, Derwent Publications Ltd., London, GB; Class E14, AN 93-261623 & JP-A-5 178 811 (MITSUI TOATSU CHEM INC) 20. Juli 1993
- DATABASE WPI Section Ch, Week 8310, Derwent Publications Ltd., London, GB; Class A25, AN 83-23241K & JP-A-58 013 557 (NIPPON POLYURETHANE KK) 26. Januar 1983

## Beschreibung

Nach phosgenfreien Verfahren, vorzugsweise durch thermische Spaltung von (cyclo)aliphatischen Polycarbaminsäureestern, erhältliche Polyisocyanatzusammensetzungen werden stabilisiert durch den Zusatz mindestens eines Stabilisators aus der Gruppe der primären Stabilisatoren (2a) als Radikalfänger und/oder Antioxidants, eines sekundären Stabilisators (2b) als Peroxidspalter und/oder Reduktionsmittel und eines sauren Stabilisators (2c) als Moderator oder eines Stabilisatorsystems aus mindestens einem primären (2a) und mindestens einem sauren Stabilisator (2c) oder mindestens einem primären (2a), mindestens einem sekundären (2b) und mindestens einem sauren Stabilisator (2c).

Organische Polyisocyanate, wie z.B. aromatische, cycloaliphatische und aliphatische di- und höherwertige Polyisocyanate können nach verschiedenartigen Verfahren hergestellt werden (Annalen der Chemie 562 (1949), Seiten 75ff). Technisch insbesondere bewährt hat sich die Herstellung von organischen Polyisocyanaten durch Phosgenierung von organischen Polyaminen zu den entsprechenden Polycarbaminsäurechloriden und deren thermische Spaltung in organische Polyisocyanate und Chlorwasserstoff, so daß zur Zeit ausschließlich dieses Herstellungsverfahren industriell genutzt wird.

Problematisch bei dieser Verfahrensweise sind der hohe Umsatz von Chlor über Phosgen und Carbaminsäurechlorid in Chlorwasserstoff, die Toxizität des Phosgens sowie die damit verbundenen kostspieligen Sicherheitsvorkehrungen, die Korrosivität der Reaktionsmischung, die Labilität der üblicherweise eingesetzten Lösungsmittel und die Bildung von chlorhaltigen und chlorfreien Nebenprodukten, die die physikalischen Eigenschaften, wie z.B. die Farbe, die Viskosität und den Dampfdruck, und chemischen Eigenschaften, wie z.B. Reaktivität, Lagerbeständigkeit u.a. der Polyisocyanate sowie die mechanischen Eigenschaften der aus solchen Polyisocyanaten hergestellten Polyisocyanat-Polyadditionsprodukten entscheidend mitbestimmen.

Es hat daher nicht an Versuchen gefehlt, organische, vorzugsweise aromatische Polyisocyanate ohne die Verwendung von Phosgen, d.h. nach phosgenfreien Verfahren, herzustellen.

Nach Angaben der EP-B-0 126 299 (US-A-4 596 678), EP-B-0 126 300 (US-A-4 596 679) und EP-A-0 355 443 (US-A-5 087 739) können (cyclo)aliphatische Diisocyanate, wie 1,6-Hexamethylen-diisocyanat (HDI) und/oder isomere aliphatische Diisocyanate mit 6 Kohlenstoffatomen im Alkylenrest und 1-Isocyanato-3-isocyanato-methyl-3,5,5-trimethyl-cyclohexan (Isophoron-diisocyanat bzw. IPDI) in Kreislaufverfahren hergestellt werden durch Umsetzung der (cyclo)aliphatischen Diamine mit Harnstoff und Alkoholen und gegebenenfalls N-unsubstituierten Carbaminsäureestern, Dialkylcarbonaten und anderen aus dem Reaktionsprozeß zurückgeführten Nebenprodukten zu (cyclo)aliphatischen Bis-carbaminsäureestern und deren thermische Spaltung in die entsprechenden Diisocyanate und Alkohole.

Je nach Art des Herstellungsverfahrens enthalten die organischen Polyisocyanate unterschiedliche Nebenprodukte mit vielfach unbekannter Struktur, wobei insbesondere die chlorhaltigen Nebenprodukte die Lagerbeständigkeit, Reaktivität und Farbe der Zusammensetzung beeinflussen.

Nach Angaben der US-A-3 330 849 können z.B. organische Polyisocyanate durch den Zusatz von Sulfonylisocyanaten gegen eine Verfärbung und Niederschlagsbildung stabilisiert werden. Durch den Zusatz von Metallnaphthenaten, wie z.B. Cadmium-, Kobalt-, Kupfer-, Blei-, Mangan- oder Zinknaphthenat, kann der hydrolysierbare Chlorgehalt von Isocyanaten gemäß US-A-3 373 182 reduziert werden. Die US-A-3 384 653 und US-A-3 449 256 beschreiben die Verbesserung der Lagerbeständigkeit von 4,4'-Diphenylmethan-diisocyanat durch eine Behandlung bei 160° bis 250°C mit Trialkylphosphaten. Der Gehalt an hydrolysierbaren Chlorverbindungen kann gemäß US-A-3 458 558 bei organischen Isocyanaten auch mit Kupfer, Silber, Nickel, Eisen und Zink bei Temperaturen über 100°C erniedrigt werden. Nach Angaben der US-A-3 479 393 stabilisieren Trialkylaminborane Isocyanate gegen eine Verfärbung. Gemäß US-A-3 535 359 eignen sich Ortho-carbonsäureester zur Stabilisierung von organischen Isocyanaten gegen eine Viskositätserhöhung. Diphenylmethan-diisocyanat enthaltende Polyisocyanatmischungen können nach Angaben der US-A-3 585 229 durch Zusatz von Diphenyldecylphosphit entfärbt werden. Gegen eine Zersetzung können organische Polyisocyanate gemäß US-A-3 692 813 mit Hilfe von Oxycarbonylisocyanaten mit mindestens einer Gruppe der Formel -O-CO-NCO stabilisiert werden. Zur Stabilisierung von organischen Polyisocyanaten gegen eine Verfärbung kann nach Angaben der US-A-3 715 381 2,6-Di-tert.-butyl-p-kresol eingesetzt werden. Diphenylmethan-diisocyanate können gemäß US-A-3 970 680 auch durch Zugabe von tertiären Aminen stabilisiert werden. Zur Reinigung von organischen Isocyanaten können diese gemäß US-A-4 065 362 bei Temperaturen über 100°C mit einem Metallsalz des Mercaptobenzthiazol, einem Metallsalz von alkylsubstituierten Dithiocarbaminsäuren, einem alkylsubstituierten Phenol, einem Thio-bisphenol oder einem Triarylphosphit behandelt werden. Nach Angaben der US-A-3 247 236 können durch Umsetzung von Diaminen mit Phosgen hergestellte und durch Destillation gereinigte Diisocyanate durch Zusatz von Kohlendioxid oder Schwefeldioxid stabilisiert werden. Nachteilig an diesem Verfahren ist die gute Löslichkeit des Schwefeldioxids im Polyisocyanat und die Ausbildung von Verfärbungen während der Lagerung. Die genannten Patentpublikationen vermitteln keine Lehre hinsichtlich der Stabilisierung von nach phosgenfreien Verfahren, vorzugsweise von durch thermische Spaltung von organischen Polycarbaminsäureestern hergestellten organischen Polyisocyanaten.

Nach phosgenfreien Verfahren, insbesondere durch thermische Spaltung von (cyclo)aliphatischen Polycarbaminsäureestern erhältliche (cyclo)aliphatische Polyisocyanate sind nicht lagerbeständig. Ihre Instabilität ist auf das Fehlen von hydrolysierbaren Chlorverbindungen und auf das Vorliegen von katalytisch wirkenden Verunreinigungen unbekannter Struktur zurückzuführen, die z.B. die Bildung von Oligomeren begünstigen. Bei tiefen Temperaturen, z.B. bei +5°C und darunter, neigt z.B. Hexamethylen-diisocyanat (HDI) zur Bildung linearer HDI-Oligomerer der Formel 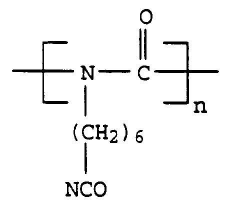
Die Molekulargewichtserhöhung, verbunden mit einer Viskositätszunahme, kann zum Gelieren des Polyisocyanats, z.B. HDI's führen. Derartige Produkte sind nur schwierig handhabbar, nicht mehr reproduzierbar zu Polyisocyanat-Polyadditionsprodukten umsetzbar und müssen daher verworfen werden. Bei höheren Lagertemperaturen nimmt z.B. die Reaktivität des nach phosgenfreien Verfahren hergestellten HDI, insbesondere bei der mit quaternären Ammoniumhydroxidverbindungen katalysierten Trimerisierungsreaktion, stark ab. Man erhält intensiv gefärbte, Isocyanuratgruppen aufweisende Polyisocyanate, die insbesondere zur Verwendung als Lackrohstoff nicht mehr verwertbar sind.

Die Aufgabe der vorliegenden Erfindung bestand darin, nach phosgenfreien Verfahren hergestellte organische, vorzugsweise (cyclo)aliphatische Polyisocyanate durch geeignete Maßnahmen zu stabilisieren, ohne die Reaktivität der Polyisocyanate nachteilig zu beeinflussen. Die Oligomerisierung der Polyisocyanate bzw. eine Viskositätserhöhung der Polyisocyanatzusammensetzung sollte ebenso verhindert werden, wie ihre Verfärbung während der Lagerung.

Diese Aufgabe konnte überraschenderweise durch den Zusatz an sich bekannter Stabilisatoren gelöst werden.

Gegenstand der Erfindung sind somit lagerstabile, organische, vorzugsweise (cyclo)aliphatische, Polyisocyanatzusammensetzungen, die enthalten oder vorzugsweise
bestehen aus
1) ein(em) nach einem phosgenfreien Verfahren hergestellten Polyisocyanat, vorzugsweise ein(em) durch thermische Spaltung von (cyclo)aliphatischen Polycarbaminsäureestern erhältliches(n) (cyclo)aliphatischen Polyisocyanat und
2) einem Stabilisator aus der Gruppe der
   2a) primären Stabilisatoren oder
   2b) sekundären Stabilisatoren oder
   2c) sauren Stabilisatoren oder
3) ein Stabilisatorsystem aus
   mindestens einem primären (2a) und mindestens einem sauren Stabilisator (2c) oder
   mindestens einem primären (2a), mindestens einem sekundären (2b) und mindestens einem sauren Stabilisator (2c).

Gegenstände der Erfindung sind ferner ein Verfahren zur Verbesserung der Stabilität von nach phosgenfreien Verfahren, vorzugsweise durch thermische Spaltung von (cyclo)aliphatischen Polycarbaminsäureestern erhältlichen Polyisocyanatzusammensetzungen gemäß einem der Ansprüche 10 bis 12 und die bevorzugte Verwendung der erfindungsgemäßen stabilen (cyclo)aliphatischen Polyisocyanatzusammensetzungen zur Herstellung von Isocyanuratgruppen enthaltenden Polyisocyanaten nach Anspruch 13.

Obgleich sich das Nebenproduktspektrum von durch thermische Spaltung von Carbaminsäurechloriden und von nach phosgenfreien Verfahren, vorzugsweise von durch thermische Spaltung von Carbaminsäureestern erhältlichen Polyisocyanaten beträchtlich unterscheidet, konnte durch den Zusatz der an sich bekannten, in Gegenwart von chlorhaltigen Nebenprodukten wirksamen Stabilisatoren überraschenderweise auch die Reaktivität und Farbzahl von nach phosgenfreien Verfahren hergestellten (cyclo)aliphatischen Polyisocyanaten stabilisiert werden. Die erfindungsgemäßen (cyclo)aliphatischen Polyisocyanatzusammensetzungen sind unter den üblichen Lagerbedingungen, z.B. bei Temperaturen im Bereich von -20 bis +40°C lagerstabil, d.h. ihre physikalischen und chemischen Kenndaten sind im wesentlichen konstant.

Die erfindungsgemäßen Polyisocyanatzusammensetzungen können beliebige (cyclo)aliphatische Polyisocyanate enthalten mit der Maßgabe, daß diese nach geeigneten Verfahren in Abwesenheit von Phosgen hergestellt wurden. Vorzüglich bewährt haben sich und daher vorzugsweise Verwendung finden (cyclo)aliphatische Polyisocyanate, die durch thermische Spaltung von (cyclo)aliphatischen Polycarbaminsäureestern erhältlich sind. Geeignete aliphatische Polyisocyanate besitzen vorteilhafterweise 3 bis 16 C-Atome, vorzugsweise 4 bis 12 C-Atome im linearen oder verzweigtkettigen Alkylenrest und geeignete cycloaliphatische Polyisocyanate vorteilhafterweise 4 bis 18 C-Atome, vorzugsweise 6 bis 15 C-Atome im Cycloalkylenrest. Beispielhaft genannt seien: 1,4-Diisocyanato-butan, 2-Ethyl-1,4-diisocyanato-butan, 1,5-Diisocyanato-pentan, 2-Methyl-1,5-diisocyanato-pentan, 2,2-Dimethyl-1,5-diisocyanato-pentan, 2-Ethyl-2-propyl-1,5-diisocyanato-pentan, 2-Ethyl-2-butyl-1,5-diisocyanato-pentan, 2-Alkoxymethylen-1,5-diisocyanato-pentan, 1,6-Hexamethylen-diisocyanat, 2,4,4- bzw. 2,2,4-Trimethyl-1,6-hexamethylen-diisocyanat, 1,7-Diisocyanatoheptan, 1,8-Diisocyanato-octan, 1,10-Diisocyanato-decan, 1,12-Diisocyanato-dodecan, 4,4'-Diisocyanato-dicyclohexylmethan, 2,4'-Diisocyanato-dicyclohexylmethan sowie Gemische der Diisocyanato-dicyclohexylmethan-Isomeren, 1,3-Diisocyanato-cyclohexan sowie Isomerengemische von Diisocyanato-cyclohexanen und 1-Isocyanato-3-isocyanatomethyl-3,5,5-trimethylcyclohexan. Als (cyclo)aliphatische Polyisocyanate vorzugsweise verwendet werden 1,6-Hexamethylen-diisocyanat, isomere aliphatische Diisocyanate mit 6 Kohlenstoffatomen im Alkylenrest sowie Mischungen davon und 1-Isocyanato-3-isocyanatomethyl-3,5,5-trimethylcyclohexan.

Wie bereits dargelegt wurde, werden die (cyclo)aliphatischen Diisocyanate vorzugsweise durch thermische Spaltung der entsprechenden Dicarbaminsäureester hergestellt. Diese Spaltung kann beispielsweise bei Temperaturen von 150 bis 300°C, vorzugsweise 180 bis 250°C und Drücken von 0,001 bis 2 bar, vorzugsweise von 1 bis 200 mbar in Abwesenheit oder vorzugsweise Gegenwart von Katalysatoren in geeigneten Spaltreaktoren, wie z.B. Dünnschicht- oder vorzugsweise Heizkerzenverdampfern nach der EP-A-0 524 554 durchgeführt werden. Die bei der Spaltung entstehenden Diisocyanate und Alkohole können beispielsweise durch fraktionierte Kondensation oder vorzugsweise durch Rektifikation getrennt und die Diisocyanate z.B. durch Destillation zusätzlich gereinigt werden.

Zur Stabilisierung der nach phosgenfreien Verfahren, vorzugsweise der durch thermische Spaltung von (cyclo)aliphatischen Polycarbaminsäureestern erhältlichen (cyclo)aliphatischen Polyisocyanate finden als primäre Stabilitsatoren (2a) zweckmäßigerweise Verbindungen Verwendung, die üblicherweise als Antioxidants und/oder Radikalfänger wirksam sind. Geeignete primäre Stabilisatoren (2a) im Sinne der Erfindung sind vorzugsweise phenolische Antioxidantien, das sind Verbindungen, die mindestens eine sterisch gehinderte phenolische Gruppierung enthalten. Als phenolische Antioxidantien beispielhaft genannt seien 2,6-Di-t-butyl-4-methylphenol, 2,4,6-Tri-t-butylphenol, 2,2'-Methylen-bis-(4-methyl-6-t-butylphenol), 2,2'-Thio-bis-(4-methyl-6-t-butylphenol), 4,4'-Thio-bis-(3-methyl-6-t-butylphenol), 4,4'-Butyliden-bis-(6-t-butyl-3-methylphenol), 4,4'-Methyliden-bis-(2,6-di-t-butylphenol), 2,2'-Methylen-bis-[4-methyl-6-(1-methylcyclohexyl)phenol], Tetrakis[methylen-3-(3,5-di-t-butyl-4-hydroxyphenyl)-propionat]methan, 1,3,5-Trimethyl-2,4,6-tris-(3,5-di-t-butyl-4-hydroxybenzyl)benzol, N,N'-Hexamethylen-bis-(3,5-di-t-butyl-4-hydroxy-hydrozimtsäureamid), Octadecyl-3-(3,5-di-t-butyl-4-hydroxyphenyl)-propionat, 1,3,5-Tris-(3,5-di-t-butyl-4-hydroxybenzyl)-isocyanurat, 1,1,3-Tris-(5-t-butyl-4-hydroxy-2-methylphenyl)butan, 1,3,5-Tris-(3,5-di-t-butyl-4-hydroxybenzyl)mesitylen, Ethylenglykol-bis[3,3-bis-(3'-t-butyl-4'-hydroxyphenyl)butyrat], 2,2'-Thiodiethyl-bis-3-(3,5-di-t-butyl-4-hydroxyphenyl)propionat, Di-(3-t-butyl-4'-hydroxy-5-methylphenyl)dicyclopentadien, 2,2'-Methylen-bis-(4-methyl-6-cyclohexylphenol), 1,6-Hexandiol-bis-(3,5-di-t-butyl-4-hydroxyphenyl)propionat, 2,4-Bis(n-octylthio)-6-(4-hydroxy-3,5-di-t-butylanilino)-1,3,5-triazin, 3,5-Di-t-butyl-4-hydroxybenzyl-phosphonsäure-diethylester und Triethylenglykol-bis-3-(t-butyl-4-hydroxy-5-methylphenyl)-propionat.

Vorzüglich bewährt haben sich und daher vorzugsweise verwendet werden z.B. Octadecyl-3-(3,5-di-t-butyl-4-hydroxyphenyl)propionat, 2,2'-Methylen-bis-(4-methyl-6-cyclohexylphenol), 2,2'-Methylen-bis-(4-methyl-6-t-butylphenol), Triethylen-glykol-bis-3-(t-butyl-4-hydroxy-5-methylphenyl)-propionat, Tetrakis[methylen-3-(3,5-di-t-butyl-4-hydroxyphenyl)-propionat]methan und insbesondere 2,6-Di-t-butyl-4-methylphenol und 3,5-Di-tert.-butyl-4-hydroxy-anisol.

Als primäre Stabilisatoren (2a) verwendet werden können ferner stickstoffhaltige Stabilisatoren, vorzugsweise mit Alkyl-, Cycloalkyl- und/oder Arylresten substituierte aromatische Amine wie z.B. 4,4'-Di-t-octyldiphenylamin, 4,4'-Di-(α,α-dimethylbenzyl)-diphenylamin, Phenyl-β-naphthylamin, N-Isopropyl-N'-phenyl-p-phenylendiamin, N-Phenyl-2-naphthylamin und/oder Phenyl-2-aminonaphthalin.

Die sterisch gehinderten Phenole und aromatischen Amine können jeweils einzeln oder in Form von Mischungen verwendet werden. Verwendet werden können ferner Mischungen aus mindestens einem sterisch gehinderten Phenol und mindestens einem aromatischen Amin.

Die primären Stabilisatoren (2a) finden vorteilhafterweise in einer Menge von 1 bis 1000 ppm, vorzugsweise 20 bis 500 ppm und insbesondere 50 bis 100 ppm, bezogen auf das Gewicht der Polyisocyanatzusammensetzung, Verwendung.

Als sekundäre Stabilisatoren (2b), die als alleinige Stabilisatoren oder vorzugsweise gemeinsam mit den primären Stabilisatoren (2a) eingesetzt werden können, finden zweckmäßigerweise Verbindungen Verwendung, die üblicherweise als Peroxidspalter und/oder Reduktionsmittel wirksam sind. Geeignete sekundäre Stabilisatoren (2b) im Sinne der Erfindung sind z.B. phosphorhaltige Verbindungen, vorzugsweise Triester der phosphorigen Säure, wie z.B. Trialkylphosphite und Triarylphosphite, und Thioether.

Als sekundäre Stabilisatoren haben sich Ester der phosphorigen Säure wie z.B. Distearylpentaerythritdiphosphit, Tris-(nonylphenyl)-phosphit, Tetrakis-(2,4-di-t-butylphenyl-4,4'-biphenylen-diphosphonit, Tris-(2,4-di-t-butylphenyl)phosphit, Neopentylglykoltriethylenglykol-diphosphit, Diisodecylpentaerythrit-diphosphit, Tristearylphosphit, Trilaurylphosphit und Triphenylphosphit, das bevorzugt verwendet wird, bewährt.

Als Beispiele für geeignete Thioether seien genannt: 2-Methyl-1-propenyl-tert.-dodecylthioether, Cyclohexylidenmethyl-n-dodecylthioether, 3-Cyclohexen-(1)-ylidenmethyl-n-octadecylthioether, 3-Cyclohexen-(1)-ylidenmethyl-n-dodecylthioether, 3-Cyclohexen-(1)-ylidenmethyl-n-octylthioether, 3-Cyclohexen-(1)-ylidenmethyl-cyclohexylthioether, 3-Methyl-(3)-cyclohexen-(1)-ylidenmethyl-n-dodecyl-thioether, 3-Cyclohexen-(1)-yliden-methyl-p-tolylthioether, 3-Cyclohexen-(1)-ylidenmethyl-benzylthioether und vorzugsweise 3-Cyclohexen-(1)-ylidenmethyl-n-dodecylthioether und 1-Hexenyl-n-dodecylthioether.

Die erfindungsgemäß geeigneten sekundären Stabilisatoren (2b) aus der Gruppe der organischen Phosphite und Thioether können ebenso wie die primären Stabilisatoren (2a) jeweils einzeln oder im Gemisch untereinander eingesetzt werden. Verwendbar sind jedoch auch Mischungen aus mindestens einem organischen Phosphit und mindestens einem Thioether.

Werden zur Stabilisierung der (cyclo)aliphatischen Polyisocyanatzusammensetzungen ausschließlich sekundäre Stabilisatoren verwendet, werden diese zweckmäßigerweise in einer Menge von 1 bis 1000 ppm, vorzugsweise 20 bis 500 ppm und insbesondere 50 bis 100 ppm, bezogen auf das Gewicht der Polyisocyanatzusammensetzung, eingesetzt.

Allein durch den Zusatz der primären Stabilisatoren (2a), die z.B. als Antioxidantien wirksam sind, wie beispielsweise der sterisch gehinderten Phenole, können die (cyclo)aliphatischen Polyisocyanatzusammensetzungen für mindestens 2 Monate bei einer Temperatur von 45°C sehr gut stabilisiert werden. Sofern die Polyisocyanatzusammensetzungen jedoch in größerem Umfange mit Luft in Kontakt kommen, kann es erforderlich sein, die zugesetzte Mengen z.B. an sterisch gehinderten Phenolen soweit zu erhöhen, daß der Grenzwert von 1000 ppm erreicht wird oder gar überschritten werden müßte. Da sterisch gehinderte Phenole in größeren Mengen jedoch nicht nur reaktivitätsstabilisierend, sondern auch reaktivitätserhöhend wirken können, kann letztlich nicht ausgeschlossen werden, daß unter bestimmten, zur Zeit noch unbekannten Voraussetzungen die Gelbildung der (cyclo)aliphatischen Polyisocyanate forciert werden kann. Insbesondere unter diesen Voraussetzungen hat sich der Zusatz von sekundären Stabilisatoren, vorzugsweise von Trialkyl- und/oder Triarylphosphiten, als äußerst vorteilhaft erwiesen. Durch den Zusatz der reduzierend wirkenden sekundären Stabilisatoren (2b) kann somit einer nichtvorhersehbaren Reaktivitätserhöhung entgegengesteuert werden. Mäßig reaktive (cyclo)aliphatische Polyisocyanatzusammensetzungen oder Einzelchargen nicht vollständig typgerechter Ware können mit binären Stabilisatorsystemen aus primären und sekundären Stabilisatoren vorzüglich stabilisiert und dadurch noch einer industriellen Verwertung zugeführt werden.

Hochreaktive (cyclo)aliphatische Polyisocyanate oder Einzelchargen mit einer abweichenden erhöhten Reaktivität können durch den Zusatz saurer Stabilisatoren (2c) stabilisiert werden. Die sauren Stabilisatoren wirken reaktivitätsmindernd und können eine unbeabsichtigte Oligomerisierung bei der Lagerung unterbinden. Vorteilhaft ist ferner, daß derartig stabilisierte Polyisocyanatzusammensetzungen trimerisiert werden können, ohne daß eine Erhöhung der Farbzahl eintritt.

Als saure Stabilisatoren (2c) kommen beispielsweise in Betracht organische Monocarbonsäuren und/oder organische Polycarbonsäuren, z.B. lineare oder verzweigte, aliphatische Monocarbonsäuren mit 1 bis 12 C-Atomen, vorzugsweise 1 bis 8 C-Atomen, die gegebenenfalls mit Halogenatomen, vorzugsweise Chloratomen und/oder Alkoxygruppen mit 1 bis 12 C-Atomen, vorzugsweise 1 bis 6 C-Atomen, insbesondere Methoxy- und/oder Ethoxygruppen substituiert sein können, wie z.B. Ameisensäure, Essigsäure, Propionsäure, 2,2-Dimethylpropionsäure, Buttersäure, Isobuttersäure, 2-Methoxybuttersäure, n-Valeriansäure, Chloressigsäure, Capronsäure, 2-Ethylhexansäure, n-Heptylsäure, n-Octylsäure, Caprylsäure und Pelargonsäure, aromatische Monocarbonsäuren mit 6 bis 12 C-Atomen, wie z.B. Benzoesäure, Toluylsäure und Napthensäure, aliphatische Polycarbonsäure mit 2 bis 12 C-Atomen, vorzugsweise 4 bis 6 C-Atomen, wie z.B. Oxalsäure, Bernsteinsäure, Maleinsäure, Fumarsäure, 2-Ethyl-bernsteinsäure, Glutarsäure, 2-Methylglutarsäure, Adipinsäure, 2-Methyl-, 2,2-Dimethyl-adipinsäure, 1,8-Octansäure, 1,10-Decansäure und 1,12-Dodecansäure, aromatische Dicarbonsäuren mit 8 bis 12 C-Atomen, wie z.B. Phthalsäure, Terephthalsäure und Isophthalsäure, Carbonsäurechloride z.B. aliphatische und aromatische Monocarbonsäurechloride, Carbonsäuremono- und -dichloride von aliphatischen und aromatischen Polycarbonsäuren vorzugsweise Dicarbonsäuren, anorganischen Säuren, wie z.B. Phosphorsäure, phosphorige Säure und Salzsäure und Diester z.B. die Alkyl- und/oder Aryl-diester der Phosphorsäure und/oder phosphorigen Säure oder anorganische Säurechloride wie z.B. Phosphoroxychlorid oder Thionylchlorid. Die sauren Stabilisatoren können einzeln oder in Form einer Mischung aus mindestens zwei sauren Stabilisatoren verwendet werden. Vorzugsweise finden als saure Stabilisatoren Verwendung aliphatische Monocarbonsäuren mit 1 bis 8 C-Atomen, wie z.B. Ameisensäure, Essigsäure und insbesondere 2-Ethyl-hexansäure und aliphatische Dicarbonsäuren mit 2 bis 6 C-Atomen, wie z.B. Oxalsäure.

Waden die sauren Stabilisatoren (2c), die auch als Moderatoren bezeichnet werden können, allein zur Stabilisierung der nach phosgenfreien Verfahren hergestellten (cyclo)aliphatischen Polyisocyanate verwendet, so werden sie zweckmäßigerweise in einer Menge von 1 bis 1000 ppm, vorzugsweise 20 bis 500 ppm und insbesondere 50 bis 100 ppm, bezogen auf das Gewicht der Polyisocyanatzusammensetzung, eingesetzt.

Die erfindungsgemäß verwendbaren primären, sekundären und sauren Stabilisatoren können, wie bereits ausgeführt wurde, einzeln zur Stabilisierung der nach phosgenfreien Verfahren, vorzugsweise durch thermische Spaltung von (cyclo)aliphatischen Polycarbaminsäureestern erhältlichen Polyisocyanaten eingesetzt werden. Aufgrund des unterschiedlichen Spektrums an Nebenprodukten und deren Mengen ha es sich jedoch als zweckmäßig erwiesen, Stabilisatorsysteme aus mindestens 2 der Stabilisatoren (2a) bis (2c) zu verwenden. Geeignete Stabilisatorsysteme können aufgrund der physikalischen Werte und einer chemischen Analyse der (cyclo)aliphatischen Polyisocyanate theoretisch und/oder experimentell ermittelt werden. Bevorzugte Anwendung finden Stabilisatorsysteme, die enthalten oder vorzugsweise bestehen aus mindestens einem primären (2a) und mindestens einem sauren Stabilisator (2c) oder mindestens einem primären (2a), mindestens einem sekundären (2b) und mindestens einem sauren Stabilisator (2c). Derartige Stabilisatorsysteme enthalten vorteilhafterweise, bezogen auf das Gewicht der Polyisocyanatzusammensetzung,
1 bis 1000 ppm, vorzugsweise 20 bis 500 ppm und insbesondere 50 bis 100 ppm des primären Stabilisators (2a)
0 bis 1000 ppm, vorzugsweise 1 bis 1000 ppm und insbesondere 50 bis 100 ppm des sekundären Stabilisators (2b) und
0 bis 1000 ppm, vorzugsweise 1 bis 1000 ppm und insbesondere 50 bis 100 ppm des sauren Stabilisators (2c).
Sehr gut bewährt haben sich und daher vorzugsweise verwendet werden Stabilisatorsysteme, die als primären Stabilisator (2a) ein sterisch gehindertes Phenol, insbesondere 2,6-Di-tert.-butyl-4-methylphenol, als sekundären Stabilisator, ein organisches Triphosphit, insbesondere Triphenylphosphit und als sauren Stabilisator (2c) eine organische Carbonsäure, insbesondere 2-Ethylhexansäure enthalten.

Zur Herstellung der erfindungsgemäßen (cyclo)aliphatischen Polyisocyanatzusammensetzung mit einer verbesserten Stabilität können die Stabilisatoren (2a) bis (2c) einzeln, nacheinander oder gleichzeitig, in fester Form, als Lösung, Dispersion und/oder Emulsion den (cyclo)aliphatischen Polyisocyanaten in der erforderlichen, wirksamen Menge einverleibt werden. Die Zugabe der Stabilisatoren erfolgt vorteilhafterweise bei Temperaturen von -10 bis 50°C, vorzugsweise von 20 bis 40°C zweckmäßigerweise unter Rühren der (cyclo)aliphatischen Polyisocyanate.

Die erfindungsgemäßen (cyclo)aliphatischen Polyisocyanatzusammensetzungen sind mehr als 12 Wochen lagerbeständig. Sie können direkt zu Polyisocyanat-Polyadditionsprodukten oder zu modifizierten Polyisocyanaten z.B. zu mit Urethan-, Allophanat-, Uretonimin-, Carbodiimid- und/oder Biuretgruppen modifizierten Polyisocyanaten, umgesetzt werden. Vorzugsweise Verwendung finden die erfindungsgemäß stabilisierten Polyisocyanatzusammensetzungen zur Herstellung von Isocyanuratgruppen enthaltenden Polyisocyanaten.

### Beispiele

### Beispiel 1

Ein durch thermische Zersetzung von Hexamethylen-dibutylurethan-1,6 hergestelltes Hexamethylen-diisocyanat-1,6 (HDI) wurde unter Rühren bei 23° mit 100 mol-ppm 2,6-Di-tert.-butyl-4-methylphenol versetzt und in einem Behälter aus V2A-Stahl unter Stickstoff bei 45°C 70 Tage lang gelagert.

### Vergleichsbeispiel I

Ein Teil des obengenannten HDI wurde unter denselben Bedingungen, jedoch in Abwesenheit eines Stabilisators gelagert.

### Herstellung von Isocyanuratgruppen enthaltenden Polyisocyanatmischungen

Die vorgenannten HDI's wurden in Gegenwart von 500 ppm N,N,N-Trimethyl-N-(2-hydroxypropyl)-ammonium-2-ethylhexanoat bei 80°C unter Rühren trimerisiert. Nach einer Reaktionszeit von einer Stunde wurde die Umsetzung durch Zugabe von Dibutylphosphat gestoppt.

An den Reaktionsmischungen wurden folgende Eigenschaften gemessen:

| | Beispiel 1 | Vergleichsbeispiel I |
|---|---|---|
| HDI-Gehalt nach der Lagerung. [Gew.-%] Restliche Bestandteile zu 100 Gew.-%: | 97,2 Oligomere | 98,2 Oligomere |
| NCO-Gehalt der Reaktionsmischung nach der Trimerisierung [Gew.-%] | 36,8 | 43,9 |
| Farbzahl der Reaktionsmischung nach der Trimerisierung [Hazen] | 54 | 316 |

Das unstabilisierte HDI des Vergleichsbeispiels I besitzt eine geringere Reaktivität, die zu einem geringeren Umsatz und zu einer deutlich schlechteren Farbzahl bei der Bildung der Isocyanuratgruppen enthaltenden Polyisocyanatmischung beiträgt.

### Beispiel 2

Ein durch thermische Zersetzung von Hexamethylen-dibutylurethan-1,6 hergestelltes Hexamethylen-diisocyanat-1,6 wurde unter Rühren bei 23°C mit 100 mol-ppm 2,6-Di-tert.-butyl-4-methylphenol und 500 mol-ppm Triphenylphosphit versetzt und in einem Behälter aus V2A-Stahl unter Stickstoff bei 45°C 80 Tage lang gelagert.

### Vergleichsbeispiel II

Ein Teil des obengenannten HDI wurde unter denselben Bedingungen, jedoch in Abwesenheit eines Stabilisators gelagert.

Die Herstellung von Isocyanuratgruppen enthaltenden Polyisocyanatmischungen erfolgte analog den Angaben des Beispiels 1 bzw. Vergleichsbeispiels I.

An den Reaktionsmischungen wurden die folgenden Eigenschaften gemessen:

| | Beispiel 2 | Vergleichsbeispiel II |
|---|---|---|
| HDI-Gehalt nach der Lagerung. [Gew.-%] Restliche Bestandteile zu 100 Gew.-%: | 95,9 Oligomere | 98,2 Oligomere |
| NCO-Gehalt der Reaktionsmischung nach der Trimerisierung [Gew.-%] | 38,2 | 41,5 |
| Farbzahl der Reaktionsmischung nach der Trimerisierung [Hazen] | 35 | 256 |

### Beispiel 3

Ein durch thermische Zersetzung von Hexamethylen-dibutylurethan-1,6 hergestelltes Hexamethylendiisocyanat-1,6 wurde unter Rühren bei 23°C mit 500 mol-ppm 2-Ethyl-hexansäure versetzt und in einem Behälter aus V2A-Stahl unter Stickstoff bei 45°C 60 Tage lang gelagert.

### Vergleichsbeispiel III

Ein Teil des obengenannten HDI wurde unter denselben Bedingungen, jedoch in Abwesenheit eines Stabilisators gelagert.

Die Herstellung von Isocyanuratgruppen enthaltenden Polyisocyanatmischungen erfolgte analog den Angaben des Beispiels 1 bzw. Vergleichsbeispiels I.

An den Reaktionsmischungen wurden folgende Eigenschaften gemessen:

| | Beispiel 3 | Vergleichsbeispiel III |
|---|---|---|
| HDI-Gehalt nach der Lagerung. [Gew.-%] Restliche Bestandteile zu 100 Gew.-%: | 98,7 Oligomere | 97,7 Oligomere |
| NCO-Gehalt der Reaktionsmischung nach der Trimerisierung [Gew.-%] | 38,0 | 40,0 |
| Farbzahl der Reaktionsmischung nach der Trimerisierung [Hazen] | 162 | 220 |

Durch den Stabilisatorzusatz wird die Oligomerbildung bei der Lagerung reduziert und eine Isocyanuratgruppen enthaltende Polyisocyanatmischung mit deutlich besserer Farbzahl erhalten. Die HDI-Reaktivität wird durch den Stabilisator nicht negativ beeinflußt.

### Beispiel 4

Ein durch thermische Zersetzung von Hexamethylen-dibutylurethan-1,6 hergestelltes Hexamethylendiisocyanat-1,6 wurde unter Rühren bei 23°C mit 100 mol-ppm 2,6-Di-tert.-butyl-4-methylphenol und 100 mol-ppm 2-Ethyl-hexansäure versetzt und in einem Behälter aus V2A-Stahl unter Stickstoff bei 45°C 47 Tage lang gelagert.

### Vergleichsbeispiel IV

Ein Teil des obengenannten HDI wurde unter denselben Bedingungen, jedoch in Abwesenheit eines Stabilisators gelagert.

Die Herstellung von Isocyanuratgruppen enthaltenden Polyisocyanatmischungen erfolgte analog den Angaben des Beispiels 1 bzw. Vergleichsbeispiels I.

An den Reaktionsmischungen wurden folgende Eigenschaften gemessen:

| | Beispiel 4 | Vergleichsbeispiel IV |
|---|---|---|
| HDI-Gehalt nach der Lagerung. [Gew.-%] Restliche Bestandteile zu 100 Gew.-%: | 98,2 Oligomere | 98,2 Oligomere |
| NCO-Gehalt der Reaktionsmischung nach der Trimerisierung [Gew.-%] | 35,9 | 40,0 |
| Farbzahl der Reaktionsmischung nach der Trimerisierung [Hazen] | 55 | 143 |

Durch die Stabilisatorkombination wird die Reaktivität des HDI's und die Farbzahl bei der Isocyanuratbildung stabilisiert.

### Beispiel 5

Ein durch thermische Zersetzung von Hexamethylen-dibutylurethan-1,6 hergestelltes Hexamethylendiisocyanat-1,6 wurde unter Rühren bei 23°C mit 100 mol-ppm 2,6-Di-tert.-butyl-4-methylphenol, 500 mol-ppm Triphenylphosphit und 100 mol-ppm 2-Ethyl-hexansäure versetzt und in einem Behälter aus V2A-Stahl unter Stickstoff bei 45°C 80 Tage lang gelagert.

### Vergleichsbeispiel V

Ein Teil des obengenannten HDI wurde unter denselben Bedingungen, jedoch in Abwesenheit eines Stabilisators gelagert.

Die Herstellung von Isocyanuratgruppen enthaltenden Polyisocyanatmischungen erfolgte analog den Angaben des Beispiels 1 bzw. Vergleichsbeispiels I.

An den Reaktionsmischungen wurden folgende Eigenschaften gemessen:

| | Beispiel 5 | Vergleichsbeispiel V |
|---|---|---|
| HDI-Gehalt nach der Lagerung. [Gew.-%] Restliche Bestandteile zu 100 Gew.-%: | 98,7 Oligomere | 98,2 Oligomere |
| NCO-Gehalt der Reaktionsmischung nach der Trimerisierung [Gew.-%] | 37,9 | 41,5 |
| Farbzahl der Reaktionsmischung nach der Trimerisierung [Hazen] | 39 | 256 |

Die Stabilisatorkombination bewirkt eine Stabilisierung der Reaktivität und Farbzahl des HDI bei der Isocyanuratbildung. Die Oligomerbildung bei der Lagerung wird reduziert.

## Patentansprüche

1. Stabile (cyclo)aliphatische Polyisocyanatzusammensetzungen, enthaltend
1) ein nach einem phosgenfreien Verfahren hergestelltes (cyclo)aliphatisches Polyisocyanat und
2) einen Stabilisator aus der Gruppe der
2a) primären Stabilisatoren oder
2b) sekundären Stabilisatoren oder
2c) sauren Stabilisatoren oder
3) ein Stabilisatorsystem aus
mindestens einem primären (2a) und mindestens einem sauren Stabilisator (2c) oder
mindestens einem primären (2a), mindestens einem sekundären (2b) und mindestens einem sauren Stabilisator (2c).

2. Stabile (cyclo)aliphatische Polyisocyanatzusammensetzungen, enthaltend
1) ein durch thermische Spaltung von (cyclo)aliphatischen Polycarbaminsäureester erhältliches (cyclo)aliphatisches Polyisocyanat und
2) einen Stabilisator aus der Gruppe der
2a) primären Stabilisatoren oder
2b) sekundären Stabilisatoren oder
2c) sauren Stabilisatoren oder
3) ein Stabilisatorsystem aus
mindestens einem primären (2a) und mindestens einem sauren Stabilisator (2c) oder
mindestens einem primären (2a), mindestens einem sekundären (2b) und mindestens einem sauren Stabilisator (2c).

3. Stabile (cyclo)aliphatische Polyisocyanatzusammensetzungen nach Anspruch 1 oder 2, enthaltend als primäre Stabilisatoren (2a) mindestens ein sterisch gehindertes Phenol, mindestens ein aromatisches Amin oder Mischungen aus mindestens einem sterisch gehinderten Phenol und mindestens einem aromatischen Amin.

4. Stabile (cyclo)aliphatische Polyisocyanatzusammensetzungen nach Anspruch 1 oder 2, enthaltend als sekundäre Stabilisatoren (2b) mindestens ein organisches Phosphit, mindestens einen Thioether oder eine Mischung aus mindestens einem organischen Phosphit und mindestens einem Thioether.

5. Stabile (cyclo)aliphatische Polyisocyanatzusammensetzungen nach Anspruch 1 oder 2, enthaltend als sauren Stabilisator (2c) mindestens eine organische Monocarbonsäure, mindestens eine organische Polycarbonsäure, mindestens eine anorganische Säure, mindestens einen Diester der Phosphorsäure oder phosphorigen Säure, mindestens ein Carbonsäurechlorid oder mindestens ein anorganisches Säurechlorid oder eine Mischung aus mindestens 2 der genannten sauren Stabilisatoren.

6. Stabile (cyclo)aliphatische Polyisocyanatzusammensetzungen nach Anspruch 1 oder 2, enthaltend, bezogen auf das Gewicht der Polyisocyanatzusammensetzung
2a) 1 bis 1000 ppm mindestens eines primären Stabilisators (2a) oder
2b) 1 bis 1000 ppm mindestens eines sekundären Stabilisators (2b) oder
2c) 1 bis 1000 ppm mindestens eines sauren Stabilisators (2c) oder
3) ein Stabilisatorsystem aus
1 bis 1000 ppm (2a),
0 bis 1000 ppm (2b) und
0 bis 1000 ppm (2c).

7. Stabile (cyclo)aliphatische Polyisocyanatzusammensetzungen nach Anspruch 1 oder 2 enthaltend
2a) als primären Stabilisator 2,6-Di-tert.-butyl-4-methylphenol,
2b) als sekundären Stabilisator Triphenylphosphit und
2c) als sauren Stabilisator 2-Ethyl-hexansäure.

8. Stabile (cyclo)aliphatische Polyisocyanatzusammensetzungen nach Anspruch 1 oder 2, enthaltend als aliphatisches Polyisocyanat (1) 1,6-Hexamethylen-diisocyanat.

9. Stabile (cyclo)aliphatische Polyisocyanatzusammensetzungen nach Anspruch 1 oder 2, enthaltend als cycloaliphatisches Polyisocyanat (1) 1-Isocyanato-3-isocyanatomethyl-3,5,5-trimethyl-cyclohexan.

10. Verfahren zur Verbesserung der Stabilität von nach einem phosgenfreien Verfahren erhältlichen (cyclo)aliphatischen Polyisocyanatzusammensetzungen, dadurch gekennzeichnet, daß man diesen in einer wirksamen Menge einen Stabilisator aus der Gruppe der primären Stabilisatoren (2a) oder sekundären Stabilisatoren (2b) oder sauren Stabilisatoren (2c) oder ein Stabilisatorsystem aus mindestens einem primären (2a) und mindestens einem sauren Stabilisator (2c) oder mindestens einem primären (2a), mindestens einem sekundären (2b) und mindestens einem sauren Stabilisator (2c) einverleibt.

11. Verfahren nach Anspruch 10, dadurch gekennzeichnet, daß die (cyclo)aliphatische Polyisocyanatzusammensetzungen durch thermische Spaltung von (cyclo)aliphatischen Polycarbaminsäureestern erhältliche (cyclo)aliphatische Polyisocyanate enthält.

12. Verfahren nach Anspruch 10 oder 11, dadurch gekennzeichnet, daß man den (cyclo)aliphatischen Polyisocyanatzusammensetzungen einverleibt
1 bis 1000 ppm mindestens eines primären Stabilisators (2a) oder
1 bis 1000 ppm mindestens eines sekundären Stabilisators (2b) oder
1 bis 1000 ppm mindestens eines sauren Stabilisators (2c) oder ein Stabilisatorsystem aus
1 bis 1000 ppm (2a),
0 bis 1000 ppm (2b) und
0 bis 1000 ppm (2c).

13. Verwendung der stabilen (cyclo)aliphatischen Polyisocyanatzusammensetzungen nach einem der Ansprüche 1 bis 9 zur Herstellung von Isocyanuratgruppen enthaltenden Polyisocyanaten.

## Claims

1. A stable (cyclo)aliphatic polyisocyanate composition containing
1) a (cyclo)aliphatic polyisocyanate prepared by a phosgene-free method and
2) a stabilizer selected from the group consisting of
2a) primary stabilizers or
2b) secondary stabilizers or
2c) acidic stabilizers or
3) a stabilizer system comprising
at least one primary (2a) and at least one acidic stabilizer (2c) or
at least one primary (2a), at least one secondary (2b) and at least one acidic stabilizer (2c)

2. A stable (cyclo)aliphatic polyisocyanate composition containing
1) a (cyclo)aliphatic polyisocyanate obtainable by thermal cleavage of (cyclo)aliphatic polycarbamates and
2) a stabilizer selected from the group consisting of
2a) primary stabilizers or
2b) secondary stabilizers or
2c) acidic stabilizers or
3) a stabilizer system comprising
at least one primary (2a) and at least one acidic stabilizer (2c) or
at least one primary (2a), at least one secondary (2b) and at least one acidic stabilizer (2c).

3. A stable (cyclo)aliphatic polyisocyanate composition as claimed in claim 1 or 2, containing, as primary stabilizers (2a), at least one sterically hindered phenol, at least one aromatic amine or a mixture of at least one sterically hindered phenol and at least one aromatic amine.

4. A stable (cyclo)aliphatic polyisocyanate composition as claimed in claim 1 or 2, containing, as secondary stabilizers (2b), at least one organic phosphite, at least one thioether or a mixture of at least one organic phosphite and at least one thioether.

5. A stable (cyclo)aliphatic polyisocyanate composition as claimed in claim 1 or 2, containing, as acidic stabilizers (2c), at least one organic monocarboxylic acid, at least one organic polycarboxylic acid, at least one inorganic acid, at least one diester of phosphoric acid or of phosphorous acid, at least one acyl chloride or at least one inorganic acid chloride or a mixture of at least 2 of the stated acidic stabilizers.

6. A stable (cyclo)aliphatic polyisocyanate composition as claimed in claim 1 or 2, containing
2a) from 1 to 1000 ppm of at least one primary stabilizer (2a) or
2b) from 1 to 1000 ppm of at least one secondary stabilizer (2b) or
2c) from 1 to 1000 ppm of at least one acidic stabilizer (2c) or
3) a stabilizer system comprising
from 1 to 1000 ppm of (2a),
from 0 to 1000 ppm of (2b) and
from 0 to 1000 ppm of (2c),
the amounts being based on the weight of the polyisocyanate composition.

7. A stable (cyclo)aliphatic polyisocyanate composition as claimed in claim 1 or 2, containing
2a) 2,6-di-tert-butyl-4-methylphenol as the primary stabilizer,
2b) triphenyl phosphite as the secondary stabilizer and
2c) 2-ethylhexanoic acid as the acidic stabilizer.

8. A stable (cyclo)aliphatic polyisocyanate composition as claimed in claim 1 or 2, containing hexamethylene 1,6-diisocyanate as the aliphatic polyisocyanate (1).

9. A stable (cyclo)aliphatic polyisocyanate composition as claimed in claim 1 or 2, containing 1-isocyanato-3-isocyanatomethyl-3,5,5-trimethylcyclohexane as the cycloaliphatic polyisocyanate (1).

10. A process for improving the stability of (cyclo)aliphatic polyisocyanate compositions obtainable by a phosgene-free method, wherein a stabilizer selected from the group consisting of primary stabilizers (2a) or secondary stabilizers (2b) or acidic stabilizers (2c) or a stabilizer system comprising at least one primary (2a) and at least one acidic stabilizer (2c) or at least one primary (2a), at least one secondary (2b) and at least one acidic stabilizer (2c) is incorporated in an effective amount in said compositions.

11. A process as claimed in claim 10, wherein the (cyclo)aliphatic polyisocyanate compositions contain (cyclo)aliphatic polyisocyanates obtainable by thermal cleavage of (cyclo)aliphatic polycarbamates.

12. A process as claimed in claim 10 or 11, wherein
from 1 to 1000 ppm of at least one primary stabilizer (2a) or
from 1 to 1000 ppm of at least one secondary stabilizer (2b) or
from 1 to 1000 ppm of at least one acidic stabilizer (2c) or
a stabilizer system comprising
from 1 to 1000 ppm of (2a),
from 0 to 1000 ppm of (2b) and
from 0 to 1000 ppm of (2c)
is incorporated in the (cyclo)aliphatic polyisocyanate compositions.

13. Use of a stable (cyclo)aliphatic polyisocyanate composition as claimed in any of claims 1 to 9 for the preparation of isocyanurate-containing polyisocyanates.

## Revendications

1. Compositions stables de polyisocyanates (cyclo)aliphatiques contenant
1) un polyisocyanate (cyclo)aliphatique préparé selon un procédé sans phosgène et
2) un stabilisant du groupe des
2a) stabilisants primaires ou
2b) stabilisants secondaires ou
2c) stabilisants acides ou
3) un système stabilisant comprenant
au moins un stabilisant primaire (2a) et au moins un stabilisant acide (2c) ou
au moins un stabilisant primaire (2a), au moins un stabilisant secondaire (2b) et au moins un stabilisant acide (2c).

2. Compositions stables de polyisocyanates (cyclo)aliphatiques contenant
1) un polyisocyanate (cyclo)aliphatique pouvant être obtenu par coupure thermique d'esters (cyclo)aliphatiques d'acides polycarbamiques et
2) un stabilisant du groupe des
2a) stabilisants primaires ou
2b) stabilisants secondaires ou
2c) stabilisants acides ou
3) un système stabilisant comprenant
au moins un stabilisant primaire (2a) et au moins un stabilisant acide (2c) ou
au moins un stabilisant primaire (2a), au moins un stabilisant secondaire (2b) et au moins un stabilisant acide (2c).

3. Compositions stables de polyisocyanates (cyclo)aliphatiques selon l'une des revendications 1 ou 2, contenant comme stabilisants primaires (2a) au moins un phénol encombré stériquement, au moins une amine aromatique ou des mélanges d'au moins un phénol encombré stériquement et d'au moins une amine aromatique.

4. Compositions stables de polyisocyanates (cyclo)aliphatiques selon l'une des revendications 1 ou 2, contenant comme stabilisants secondaires (2b) au moins un phosphite organique, au moins un thioéther ou un mélange d'au moins un phosphite organique et d'au moins un thioéther.

5. Compositions stables de polyisocyanates (cyclo)aliphatiques selon l'une des revendications 1 ou 2, contenant comme stabilisant acide (2c) au moins un acide monocarboxylique organique, au moins un acide polycarboxylique organique, au moins un acide inorganique, au moins un diester d'acide phosphorique ou d'acide phosphoreux, au moins un chlorure d'acide carboxylique ou au moins un chlorure d'acide inorganique ou un mélange d'au moins 2 des stabilisants acides susdits.

6. Compositions stables de polyisocyanates (cyclo)aliphatiques selon l'une des revendications 1 ou 2, contenant, par rapport au poids de la composition de polyisocyanates
2a) 1 à 1000 ppm d'au moins un stabilisant primaire (2a) ou
2b) 1 à 1000 ppm d'au moins un stabilisant secondaire (2b) ou
2c) 1 à 1000 ppm d'au moins un stabilisant acide (2c) ou
3) un système stabilisant comprenant
1 à 1000 ppm de (2a),
0 à 1000 ppm de (2b) et
0 à 1000 ppm de (2c).

7. Compositions stables de polyisocyanates (cyclo)aliphatiques selon l'une des revendications 1 ou 2, contenant
2a) comme stabilisant primaire du 2,6-di-tert-butyl-4-méthylphénol,
2b) comme stabilisant secondaire du triphénylphosphite et
2c) comme stabilisant acide de l'acide 2-éthyl-hexanoïque.

8. Compositions stables de polyisocyanates (cyclo)aliphatiques selon l'une des revendications 1 ou 2, contenant comme polyisocyanate aliphatique (1) du 1,6-hexaméthylène-diisocyanate.

9. Compositions stables de polyisocyanates (cyclo)aliphatiques selon l'une des revendications 1 ou 2, contenant comme polyisocyanate cycloaliphatique (1) du 1-isocyanato-3-isocyanatométhyl-3,5,5-triméthyl-cyclohexane.

10. Procédé pour l'amélioration de la stabilité de compositions de polyisocyanates (cyclo)aliphatiques pouvant être obtenues selon un procédé sans phosgène, caractérisé par le fait qu'on incorpore à celles-ci, en une quantité efficace, un stabilisant choisi dans le groupe des stabilisants primaires (2a) ou des stabilisants secondaires (2b) ou des stabilisants acides (2c) ou un système stabilisant comprenant au moins un stabilisant primaire (2a) et au moins un stabilisant acide (2c) ou au moins un stabilisant primaire (2a), au moins un stabilisant secondaire (2b) et au moins un stabilisant acide (2c).

11. Procédé selon la revendication 10, caractérisé par le fait que les compositions de polyisocyanates (cyclo)aliphatiques contiennent des polyisocyanates (cyclo)aliphatiques pouvant être obtenus par coupure thermique d'esters (cyclo)aliphatiques d'acides polycarbamiques.

12. Procédé selon l'une des revendications 10 ou 11, caractérisé par le fait qu'on incorpore aux compositions de polyisocyanates (cyclo)aliphatiques
1 à 1000 ppm d'au moins un stabilisant primaire (2a) ou
1 à 1000 ppm d'au moins un stabilisant secondaire (2b) ou
1 à 1000 ppm d'au moins un stabilisant acide (2c) ou
un système de stabilisants comprenant
1 à 1000 ppm de (2a),
0 à 1000 ppm de (2b) et
0 à 1000 ppm de (2c).

13. Utilisation des compositions de polyisocyanates (cyclo)aliphatiques selon l'une quelconque des revendications 1 à 9 pour la préparation de polyisocyanates contenant des groupes isocyanurate.
